# EUROPEAN PATENT SPECIFICATION

(11) **EP 1 882 474 B1**
(45) Date of publication and mention of the grant of the patent: **03.11.2010**
(21) Application number: 06732351.9
(22) Date of filing: 26.04.2006
(51) Int. Cl.: A61K 31/496, A61P 3/06, A61P 3/10, C07D 417/14

(54) **PROPHYLACTIC/THERAPEUTIC AGENT FOR ABNORMALITIES OF LIPID METABOLISM**
PROPHYLAKTISCHES/THERAPEUTISCHES MITTEL FÜR ABWEICHUNGEN IM FETTSTOFFWECHSEL
AGENT PROPHYLACTIQUE/THERAPEUTIQUE CONCERNANT DES ANOMALIES DU METABOLISME LIPIDE

(30) Priority: 26.04.2005 JP 2005127523
(43) Date of publication of application: 30.01.2008
(73) Proprietor: Mitsubishi Tanabe Pharma Corporation, Osaka 541-8505 (JP)
(72) Inventor: ABE, Yuji, OSAKA 541-8505 (JP); KUSUNOKI, Aki, OSAKA 541-8505 (JP); HAYASHI, Yoshiharu, OSAKA 541-8505 (JP); AKAHOSHI, Fumihiko, OSAKA 541-8505 (JP)
(74) Representative: von Kreisler Selting Werner
(86) International application number: PCT/JP2006/308695
(87) International publication number: WO 2006/118127

(56) References cited:
- EP-A- 1 308 439
- EP-A- 1 854 795
- WO-A1-02/14271
- WO-A1-02/051836
- WO-A1-03/055881
- WO-A2-03/101958
- WO-A2-2005/012249
- JP-A- 2001 510 442
- JP-A- 2005 505 531
- FONG T M: "Targeting metabolic syndrome" EXPERT OPINION ON INVESTIGATIONAL DRUGS 200409 GB, vol. 13, no. 9, September 2004 (2004-09), pages 1203-1206, XP002480452 ISSN: 1354-3784
- GRIFMAN M: "Metabolic syndrome: 'The whole is greater than the sum of its parts'" DRUG DISCOVERY TODAY: TARGETS 200408 GB, vol. 3, no. 4, August 2004 (2004-08), pages 134-135, XP002480453 ISSN: 1741-8372
- STEFANOVIC V ET AL: "PLASMA CELL MEMBRANE GLYCOPROTEIN 1 (PC-1): A MARKER OF INSULIN RESISTANCE IN OBESITY, UREMIA AND DIABETES MELLITUS" CLINICAL LABORATORY, CLIN LAB PUBLICATIONS, HEIDELBERG, vol. 50, no. 5/06, 1 January 2004 (2004-01-01), pages 271-278, XP009057018 ISSN: 1433-6510
- CONARELLO S.L. ET AL.: 'Mice lacking dipeptidyl peptidase IV are protected against obesity and insulin resistance' PROC. NATL. ACAD. SCI., USA vol. 100, no. 11, 27 May 2003, pages 6825 - 6830, XP003003538
- TAKASAKI K. ET AL.: 'Effects of Combination Treatment with Dipeptidyl Peptidase IV Inhibitor and Sulfonylurea on Glucose Levels in Rats' J. PHARMACOL. SCI. vol. 95, no. 2, June 2004, pages 291 - 293, XP007900710
- SUDRE B., ET AL: 'Chronic Inhibition of Circulating Dipeptidyl Peptidase IV by FE 999011 Delays the Occurrence of Diabetes in Male Zucker Diabetic Fatty Rats' DIABETES vol. 51, no. 5, May 2002, pages 1461 - 1469, XP002994152

## Description

### Technical Field

The present invention relates to a pharmaceutical agent for the treatment and/or prophylaxis of postprandial hyperlipidemia.

### Background Art

Hyperlipidemia refers to a condition where cholesterol or neutral fat in the blood has abnormally increased, which is one of the important risk factors of the onset of arteriosclerotic diseases such as ischemic heart disease and the like.

In recent years, it has been reported that, in addition to the increased blood lipid level, diabetes and hyperinsulinemia are important risk factors of the onset of arteriosclerotic diseases (see, non-patent reference 1). Particularly, when plural risk factors such as abnormal lipid metabolism, abnormal glucose metabolism, obesity, hypertension and the like are observed, the risk of arteriosclerotic disease of the subject is considered to increase strikingly. The pathology associated with plural risk factors is attracting attention as "metabolic syndrome" or "multiple risk factor syndrome".

As the diagnostic criteria of metabolic syndromes, several healthcare organizations have proposed diagnostic criteria in recent years, such as those of World Health Organization (WHO), US treatment guideline for hyperlipidemia and the like.

For example, according to the diagnostic criteria of WHO, when a subject shows at least one of type 2 diabetes, impaired glucose tolerance and insulin resistance, and falls under at least two of an increased blood pressure (≥160/90 mmHg), increased plasma neutral fat (not less than 150 mg/dL and/or HDL cholesterol low value of less than 35 mg/dL for male, less than 39 mg/dL for female), central obesity (the ratio of waist to hip exceeding 0.90 for male, exceeding 0.85 for female and/or BMI exceeding 30 kg/m²), and a trace amount of albumin urine (urinary albumin excretion rate of not less than 20 µg/min, or the ratio of albumin:creatinine of not less than 30 mg/g), the subject is diagnosed to have a metabolic syndrome (see, non-patent reference 2).

According to US treatment guidelines for hyperlipidemia (NCEP -ATPIII: National Cholesterol Education Program Adult Treatment Panel III), when a subject falls under at least three of visceral fat type obesity (waist size exceeding 102 cm for male, 88 cm for female), increased neutral fat (blood triglyceride (hereinafter to be referred to as TG) concentration of not less than 150 mg/dL), decrease in HDL cholesterol (less than 40 mg/dL for male, less than 50 mg/dL for female), blood pressure increase (systolic blood pressure is not less than 130 mmHg, or diastolic blood pressure is not less than 85 mmHg), and blood glucose increase (fasting blood sugar level is not less than 110 mg/dL), the subject is diagnosed with a metabolic syndrome (see, non-patent reference 3).

While diagnostic criteria of metabolic syndrome partly differ between WHO and US guideline for hyperlipidemia, they are common in that obesity, hypertension, borderline diabetes, hypertriglyceridemia and low high-density lipoprotein cholesterol are important risk factors. Therefore, for the prophylaxis or treatment of arteriosclerotic diseases, it is important to control LDL cholesterol to an adequate level as well as comprehensively manage risk factors because, in metabolic syndrome, for example, abnormal lipid metabolism and abnormal glucose metabolism are observed in combination.

The management goal of blood lipid level varies depending on the presence or absence of previous ischemic heart disease and the presence or absence of risk factors (complications of hypertension, diabetes etc.) other than lipid. When most strict treatment is required, the total cholesterol is not more than 200 mg/dL, LDL cholesterol is not more than 100 mg/dL, HDL cholesterol is not less than 40 mg/dL, and TG is not more than 150 mg/dL (see, non-patent reference 3).

The main characteristics of pathologically abnormal glucose and lipid metabolism represented by a metabolic syndrome and the like are an increase in neutral fat and blood glucose levels after eating, which are called postprandial hyperlipidemia and postprandial hyperglycemia, respectively. The main blood lipid that increases after eating is TG. Consequently, VLDL rich in TG increases in blood, decreases HDL and increases the risk of arteriosclerosis (see, non-patent reference 4). Moreover, postprandial hyperlipidemia and postprandial hyperglycemia independently and additively cause oxidative stress in the vascular endothelium, increasing the risk of arteriosclerosis (see, non-patent reference 5).

While a lipid-lowering drug or a blood glucose-lowering drug is used for the treatment of abnormal glucose and lipid metabolism, the effects of these pharmaceutical agents are not entirely satisfactory. For example, while HMG-CoA reductase inhibitor affords a superior LDL cholesterol-lowering effect, it offers little hope for a blood glucose level-improving effect. In addition, while insulin sensitizer affords a good influence on blood glucose and TG, it adversely influences cardiac failure because it causes body weight gain and edema. In consideration of the above, careful medication management is demanded (see, non-patent reference 6).

A report has documented that a rapid-acting insulin secretagogue, nateglinide [(-)-N-(trans-4-isopropylcyclohexylcarbonyl)-D-phenylalanine], suppresses increase in blood lipid by fat loading in rats with type 2 diabetes (see, non-patent reference 7). However, since insulin secretagogue such as nateglinide possibly causes hypoglycemia, it requires strict medication management in line with the mealtime. Therefore, the drug is not necessary a satisfactory treatment method of postprandial hyperlipidemia and postprandial hyperglycemia.

Glucagon-like peptide-1 (hereinafter to be referred to as GLP-1) and glucose-dependent insulinotropic polypeptide (hereinafter to be referred to as GIP), which are secreted from the gastrointestinal tract after eating, have a strong insulin secretagogue action. However, since GLP-1 and GIP are degraded by dipeptidyl peptidase IV (hereinafter to be referred to as DPP-IV), they may not be able to sufficiently act in the body. DPP-IV inhibitor promotes secretion of insulin by suppressing degradation of GLP-1 and GIP and shows a hypoglycemic action. Therefore, the development thereof as a therapeutic drug for type 2 diabetes is ongoing (see, non-patent reference 8). Nevertheless, an abnormal lipid metabolism-improvement effect based on DPP-IV inhibitory action is not developed actively,

non-patent reference 1: Isomaa B, Almgren P, Tuomi T, Forsen B, Lahti K, Nissen M, Taskinen M.R., Groop L: Diabetes Care 2001; 24: 683-689.
non-patent reference 2: Alberti K.G., Zimmet P.Z.: Diabet Med 1998; 15: 539.
non-patent reference 3: National Institutes of Health: Third Report of the National Cholesterol Education Program Expert Panel on Detection, Evaluation, and Treatment of High Blood Cholesterol in Adults (Adult Treatment Panel III). Executive Summary. Bethesda, MD, National Institutes of Health, National Heart, Lung and Blood Institute, 2001 (NIH publ. no. 01-3670) non-patent reference 4: Carr, M.C., Brunzell, J.D.: J Clin Endoclinol Metab Circ 2004; 89: 2601-2607.
non-patent reference 5: Ceriello A., Taboga C., Tonutti L., Quagliaro L., Piconi L., Bais B., Ros R.D., Motz E.: Circulation 2002; 106: 1211-1218.
non-patent reference 6: Nesto R.W., Bell D., Bonow R.O., Fonseca V., Grundy S.M., Horton E.S., Winter M.L., Porte D., Semenkovich C.F., Smith S., Young L.H., Kahn R.: Circulation 2003; 108: 2941-2948.
non-patent reference 7: Mine T., Miura K., Kitahara Y., Okano A., Kawamori R.: Biol Pharm Bull. 2002; 25: 1412-1416. non-patent reference 8: Weber A.E.: J. Med. Chem. 2004; 47: 4135-4141.

EP-A-1308439, example 222 discloses the use of DPP-IV inhibitors e.g. the trihydrochloride of 3-{(2S,4S)-4-[4-(3-methyl-1-phenyl-1H-pyrazol-5-yl)piperazin-1-yl]pyrrolidin-2-ylcarbonyl}thiazolidinein the treatment of diseases such as diabetes and obesity.

FONG T M: "Targeting metabolic syndrome" EXPERT OPINION ON INVESTIGATIONAL DRUGS 200409 GB, vol. 13, no. 9, September 2004 (2004-09), pages 1203-1206, XP002480452 ISSN: 1354-3784 discloses that DPP-IV inhibitors reduce HbAlc, free fatty acids and LDL cholesterol levels in db/db-mice (= model for diabetic dyslipidemia).

GRIFMAN M: "Metabolic syndrome: 'The whole is greater than the sum of its parts "' DRUG DISCOVERY TODAY: TARGETS 200408 GB, vol. 3, no. 4, August 2004 (2004-08), pages 134-135, XP002480453 ISSN: 1741-8372 discloses that the inhibition of DPP-IV leads to satiety and weight loss.

CONARELLO S.L. ET AL.: 'Mice lacking dipeptidyl peptidase IV are protected against obesity and insulin resistance' PROC. NATL. ACAD. SCI., USA vol. 100, no. 11, 27 May 2003, pages 6825 - 6830, XP003003538 relates to the function of dipeptidyl peptidase IV in mice and suggests the use of DPP-IV inhibitors in the treatment of metabolic disorders related to both, diabetes and obesity.

WO-A-03/101958 relates to the use of DPP-IV inhibitors in the treatment of metabolic syndrome, diabetes, obesity, hyperlipidemia, etc.

STEFANOVIC V ET AL: "PLASMA CELL MEMBRANE GLYCOPROTEIN 1 (PC-1): A MARKER OF INSULIN RESISTANCE IN OBESITY, UREMIA AND DIABETES MELLITUS" CLINICAL LABORATORY, CLIN LAB PUBLICATIONS, HEIDELBERG, vol. 50, no. 5/06, 1 January 2004 (2004-01-01), pages 271-278, XP009057018 ISSN: 1433-6510 discloses that DPP-IV inhibitors improve insulin sensitivity and thus metabolic disorders related to diabetes and obesity.

WO-A-03/055881 relates to the use of DPP-IV inhibitors in the treatment of diabetes, metabolic syndrome etc.

### Disclosure of the Invention

### Problems to be Solved by the Invention

The problem of the present invention is to provide a pharmaceutical agent for the prophylaxis and/or treatment of abnormal lipid metabolism, for which a sufficient treatment method and a therapeutic drug have not been found, particularly, abnormal lipid metabolism associated with eating , and, in particular, postprandial hyperlipidemia.

### Means of Solving the Problems

The present inventors have studied in view of the above-mentioned problems and found that 3-{(2S,4S)-4-[4-(3-methyl-1-phenyl-1H-pyxazol-5-yl)piperazin-1-yl]pyrrolidin-2-ylcarbonyl}thiazolidine hydrobromide (hereinafter to be also referred to as compound 1) suppresses an increase in the plasma TG concentration after fat loading in obese Zucker fatty rat having insulin resistance (hereinafter to be referred to as ZF rat). They have studied the above result in more depth and found that, in addition to TG, the blood glucose level also increases when fat is loaded, and that compound 1 suppresses blood glucose increase after the fat loading, and further, markedly suppresses blood glucose increase after glucose loading in an oral glucose loading test after fat loading. Moreover, they have found that, unlike insulin secretagogue, compound 1 does not induce hypoglycemia, which resulted in the completion of the present invention.

### Effect of the Invention

The pharmaceutical agent of the present invention can simultaneously suppress a postprandial increase of blood TG and glucose observed in metabolic syndrome and the like with a single pharmaceutical agent. Unlike insulin secretagogue (e.g., nateglinide) and the like, the pharmaceutical agent of the present invention can be used safely without causing hypoglycemia. Moreover, it can be easily used in combination with other agents, and can correct abnormal lipid metabolism by a combined use of the pharmaceutical agent of the present invention and a general lipid-lowering drug, even when a decrease in lipid and blood glucose cannot be expected with a general lipid-lowering drug alone.
That is, the compound is effective as a pharmaceutical agent for the prophylaxis and/or treatment of abnormal lipid metabolism associated with diet, that is, postprandial hyperlipidemia.

### Brief Description of the Drawings

Fig. 1 shows the suppressive action of compound 3 on an increase in plasma TG after oral fat loading in ZF rat, where the plot at each time point shows mean±standard error. * P<0.05, ** P<0.01: comparison with vehicle group (Student's t-test)

Fig. 2 shows the suppressive action of compound 3 on an increase in plasma free fatty acids after oral fat loading in ZF rat, where the plot at each time point shows average value ±standard error. * P<0.05, ** P<0.01: comparison with vehicle group (Student's t-test)

Fig. 3 shows the suppressive action of compound 3 on an increase in plasma glucose after oral fat loading in ZF rat, where the plot at each time point shows mean±standard error. * P<0.05, ** P<0.01: comparison with vehicle group (Student's t-test)

Fig. 4 shows the action of compound 3 on the concentration of plasma insulin after oral fat loading in ZF rat, where the plot at each time point shows mean±standard error. ** P<0.01: comparison with vehicle group (Student's t-test)

### Best Mode for Embodying the Invention

That is, the present invention relates to
(1) a pharmaceutical agent comprising a salt of 3-{(2S,4S)-4-[4-(3-methyl-1-phenyl-1H-pyrazol-5-yl)piperazin-1-yl]pyrrolidin-2-ylcarbonyl}thiazolidine with 2.0 hydrobromic acid, 2.5 hydrobromic acid, 2 maleic acid, 2 tosyl acid, 2.5 hydrochloric acid, 2 naphthalene-1-sulfonic acid, 2 mesyl acid, 3 mesyl acid or 2 naphthalene-2-sulfonic acid, or a solvate thereof, for use in the prophylaxis and/or treatment of postprandial hyperlipidemia.
(2) the pharmaceutical agent of the above (1) comprising 3-{(2S,4S)-4-[4-(3-methyl-1-phenyl-1H-pyrazol-5-yl)piperazin-1-yl]pyrrolidin-2-ylcarbonyl}thiazolidine 2.5 hydrobromide, or a solvate thereof, for use in the prophylaxis and/or treatment of postprandial hyperlipidemia.

While the definitions of the therms used in the present specification are described in the following, the following definitions do not limit the scope of the present invention.

The "solvate" is a compound wherein a solvent is bonded. When the solvent is water, it may be particularly indicated as a hydrate. The salt as an active ingredient in the pharmaceutical agent of the present invention may be present as any solvate, and a hydrate is more preferable.

The abnormal lipid metabolism" means a condition where some abnormality occurs in the carbohydrate or lipid metabolism pathway (including absorption), and the blood concentration is not maintained in an appropriate range (mostly beyond the normal blood concentration range). It is a pathological state requiring a treatment according to the diagnostic criteria such as US hyperlipidemia guideline, WHO guideline and the like. Specifically, postprandial hyperlipidemia is mentioned.

The "3-{(2S,4S)-4-[4-(3-methyl-1-phenyl-1H-pyrazol-5-yl)piperazin-1-yl]pyrrolidin-2-ylcarbonyl}thiazolidine (hereinafter to be referred to as compound 2)" is a compound represented by the following chemical formula (2).

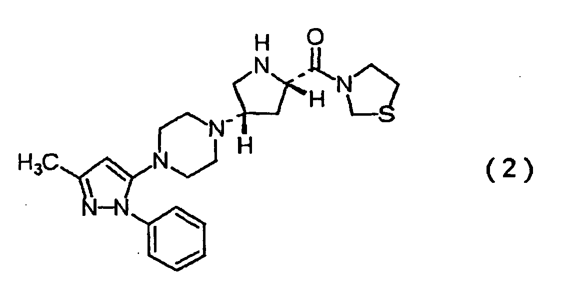

The 3 hydrochloride of compound 2 can be produced according to the synthesis method described as Example 222 of WO-A-02/14271. In addition, this can be converted to compound 2 using a suitable base.

A salt of compound 2 with the organic or inorganic mono- or di-basic acid, and a solvate thereof, which are active ingredients of the pharmaceutical agent used according to the present invention, are various salt forms of compound 2 described in the above-mentioned patent description, which are afforded according to a conventional method.

The pharmaceutical agent of the present invention can be administered orally or parenterally (intravenously, subcutaneously etc.) in a general administration form (tablet, capsule, powder etc.). The pharmaceutical agent of the present invention is desirably administered once a day or several times a day in consideration of in vivo stability and bioavailability. Such dose range is 0.01 mg - 100 mg per 1 kg of body weight.

The present invention is explained in detail in the following by referring to Experimental Examples, which are not to be construed as limitative. The "compound 3" used in the following Experimental Examples is a hydrate of 2.5 hydrobromide of the aforementioned compound 2.

### Examples

### Experimental Example 1: Effect of compound 3 on abnormal lipid metabolism and abnormal glucose metabolism after oral fat loading in ZF rat

### (Test method)

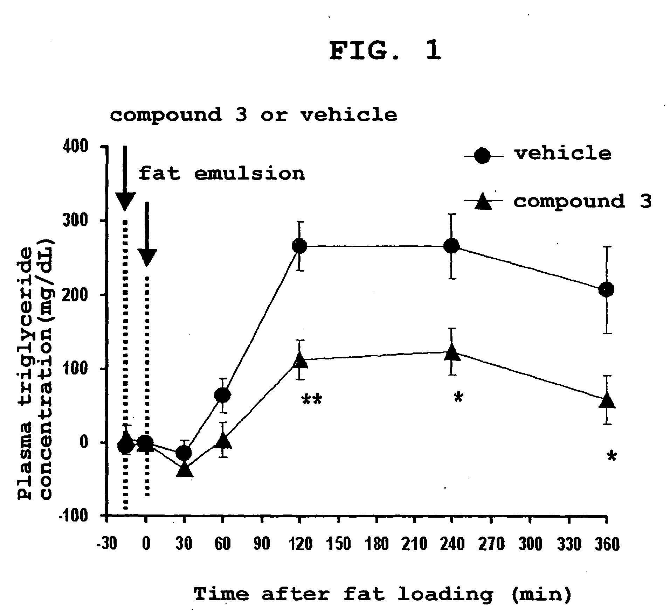

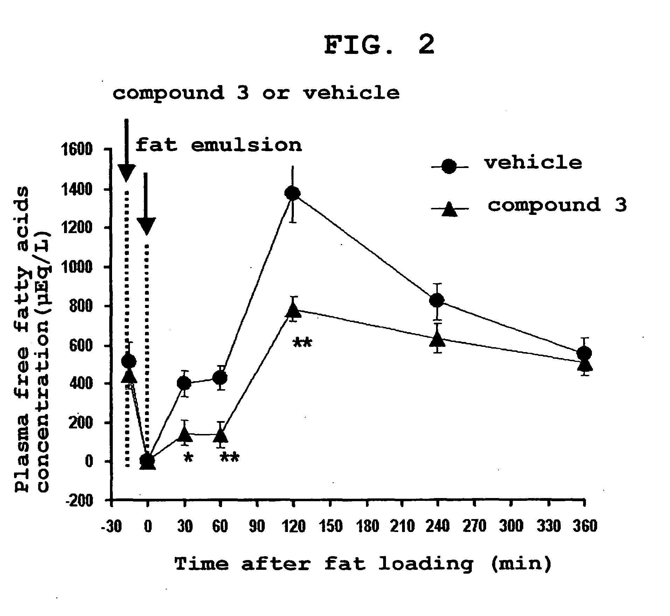

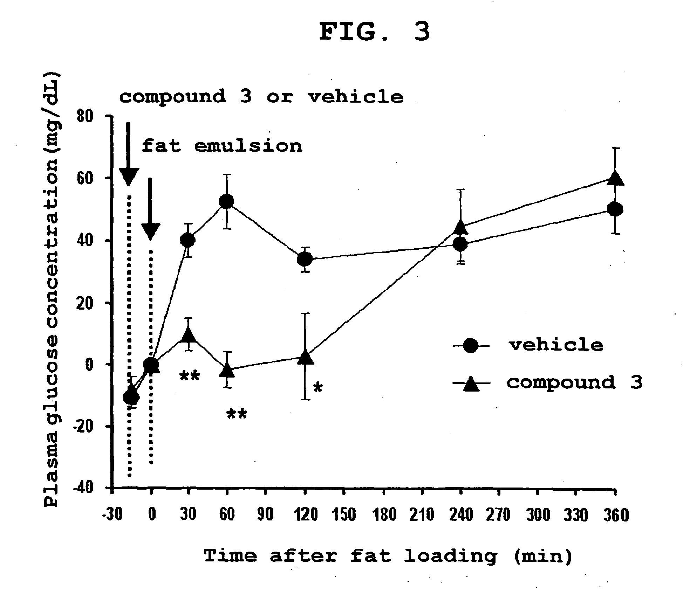

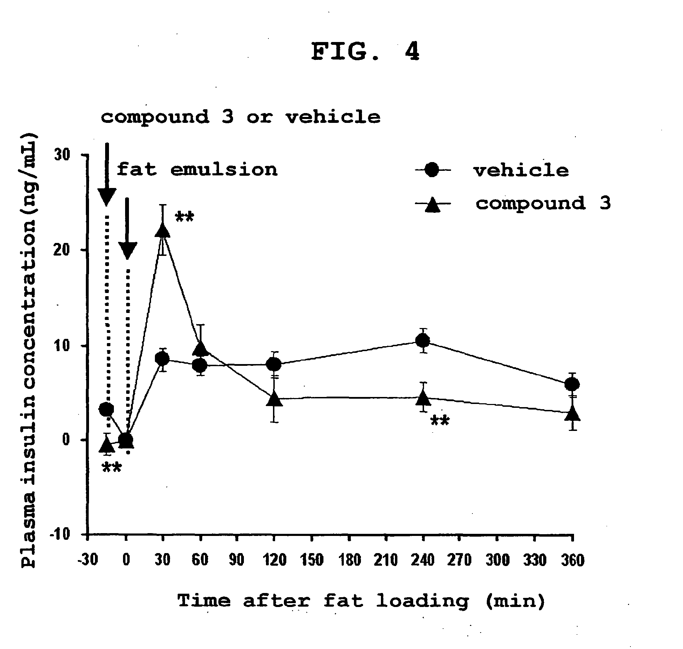

The test was performed using male ZF rats. The rats were divided into two groups (10 rats/group). Compound 3 (1 mg/kg) or a 0.5% hydroxypropylmethylcellulose solution, which was a vehicle used to dissolve the compound, was administered by gavage to each of the rats. The administered volume was 2 mL/kg for both. At 15 min after the administration, a fat emulsion (main component was soybean oil, Intralipos; Otsuka Pharmaceutical Factory, Inc.) was orally loaded at the rate of 10 mL/kg. Blood samples were sequentially collected, and plasma TG concentration, free fatty acids concentration, glucose concentration and insulin concentration were measured. The amount of change from the value before fat loading in each index is shown in Fig. 1 to Fig. 4.

### (Results)

In ZF rat, the plasma TG concentration continuously increased until 6 hr after fat loading. Compound 3 suppressed an increase in the plasma TG concentration and free fatty acids concentration after fat loading. In addition, an increase in the concentration of plasma glucose by fat loading was observed in ZF rat. Compound 3 also suppressed the increase in the plasma glucose concentration after fat loading. Moreover, compound 3 transiently increased insulin concentration after fat loading.

### Industrial Applicability

A pharmaceutically acceptable salt and the like of 3-{(2S,4S)-4-[4-(3-methyl-1-phenyl-1H-pyrazol-5-yl)piperazin-1-yl]pyrrolidin-2-ylcarbonyl}thiazolidine are effective as agents for the treatment and/or prophylaxis of abnormal lipid metabolism associated with eating and promote the development of pharmaceutical products, in particular postprandial hyperlipidemia.

## Claims

1. A pharmaceutical agent comprising a salt of 3-{(2S,4S)-4-[4-(3-methyl-1-phenyl-1H-pyrazol-5-yl)piperazin-1-yl]pyrrolidin-2-ylcarbonyl}thiazolidine with 2.0 hydrobromic acid, 2.5 hydrobromic acid, 2 maleic acid, 2 tosyl acid, 2.5 hydrochloric acid, 2 naphthalene-1-sulfonic acid, 2 mesyl acid, 3 mesyl acid or 2 naphthalene-2-sulfonic acid, or a solvate thereof, for use in the prophylaxis and/or treatment of postprandial hyperlipidemia.

2. The pharmaceutical agent according to claim 1 comprising 3-{(2S,4S)-4-[4-(3-methyl-1-phenyl-1H-pyrazol-5-yl)piperazin-1-yl]pyrrolidin-2-ylcarbonyl}thiazolidine 2.5 hydrobromide, or a solvate thereof, for use in the prophylaxis and/or treatment of postprandial hyperlipidemia.

## Patentansprüche

1. Pharmazeutisches Mittel, das ein Salz von 3-{(2S,4S)-4-[4-(3-Methyl-1-phenyl-1H-pyrazol-5-yl)piperazin-1-yl]pyrrolidin-2-ylcarbonyl}thiazolidin mit 2,0 Bromwasserstoffsäure, 2,5 Bromwasserstoffsäure, 2 Maleinsäure, 2 Tosylsäure, 2,5 Chlorwasserstoffsäure, 2 Naphthalin-1-sulfonsäure, 2 Mesylsäure, 3 Mesylsäure oder 2 Naphthalin-2-sulfonsäure oder ein Solvat davon umfasst, zur Verwendung bei der Prophylaxe und/oder Behandlung von postprandialer Hyperlipidämie.

2. Pharmazeutisches Mittel gemäß Anspruch 1, das 3-{(2S,4S)-4-[4-(3-Methyl-1-phenyl-1H-pyrazol-5-yl)piperazin-1-yl]pyrrolidin-2-ylcarbonyl}-thiazolidin-2,5-Hydrobromid oder ein Solvat davon umfasst, zur Verwendung bei der Prophylaxe und/oder Behandlung von postprandialer Hyperlipidämie.

## Revendications

1. Agent pharmaceutique comprenant un sel de 3-{(2S,4S)-4-[4-(3-méthyl-1-phényl-1H-pyrazol-5-yl)pipérazin-1-yl]pyrrolidin-2-ylcarbonyl}thiazolidine avec de 2,0 acide bromhydrique, 2,5 acide bromhydrique, 2 acide maléique, 2 acide tosylique, 2,5 acide chlorhydrique, 2 acide naphtalène-1-sulfonique, 2 acide mésylique, 3 acide mésylique ou 2 acide naphtalène-2-sulfonique, ou un solvate de celui-ci, pour utilisation dans la prophylaxie et/ou le traitement d'une hyperlipidémie post-prandiale.

2. Agent pharmaceutique selon la revendication 1, comprenant du 3-{(2S,4S)-4-[4-(3-méthyl-1-phényl-1H-pyrazol-5-yl)pipérazin-1-yl]pyrrolidin-2-yl-carbonyl}thiazolidine 2,5 bromhydrate, ou un solvate de celui-ci, pour utilisation dans la prophylaxie et/ou le traitement d'une hyperlipidémie post-prandiale.
